# EUROPEAN PATENT SPECIFICATION

(11) **EP 2 817 423 B1**
(45) Date of publication and mention of the grant of the patent: **31.05.2017**
(21) Application number: 13752350.2
(22) Date of filing: 21.02.2013
(51) Int. Cl.: C12Q 1/70, G01N 33/543

(54) **METHODS AND SYSTEMS FOR SIGNAL AMPLIFICATION OF BIOASSAYS**
VERFAHREN UND SYSTEME ZUR SIGNALVERSTÄRKUNG VON BIOASSAYS
PROCÉDÉS ET SYSTÈMES POUR L'AMPLIFICATION DE SIGNAUX DE TESTS BIOLOGIQUES

(30) Priority: 21.02.2012 US 201261601244 P
(43) Date of publication of application: 31.12.2014
(73) Proprietor: Laboratory Corporation of America Holdings, Burlington, North Carolina 27215 (US)
(72) Inventor: ANDERSON, Dwight Lyman, Minneapolis Minnesota 55406 (US); CONRAD, Andrew J., Malibu California 90265 (US); ERICKSON, Stephen Eric, White Bear Township Minnesota 55110 (US); HOPKINS, Ben Barrett, Sherman Oaks, California 91423 (US)
(74) Representative: Donald, Jenny Susan
(86) International application number: PCT/US2013/027060
(87) International publication number: WO 2013/126522

(56) References cited:
- WO-A1-2004/009848
- WO-A2-03/104424
- US-A1- 2004 137 430
- US-A1- 2006 094 076
- US-A1- 2006 094 076
- GALIKOWSKA E ET AL: "Specific detection ofandstrains by using ELISA with bacteriophages as recognition agents", EUROPEAN JOURNAL OF CLINICAL MICROBIOLOGY & INFECTIOUS DISEASES, SPRINGER, BERLIN, DE, vol. 30, no. 9, 15 February 2011 (2011-02-15), pages 1067-1073, XP019939681, ISSN: 1435-4373, DOI: 10.1007/S10096-011-1193-2
- GUTIERREZ R ET AL: "MONOCLONAL ANTIBODIES AND AN INDIRECT ELISA FOR DETECTION OF PSYCHROTROPHIC BACTERIA IN REFRIGERATED MILK", JOURNAL OF FOOD PROTECTION, INTERNATIONAL ASSOCIATION FOR FOOD PROTECTION, US, vol. 60, no. 1, 1 January 1997 (1997-01-01), pages 23-27, XP002952104, ISSN: 0362-028X

## Description

This application claims priority to U.S. Provisional Patent Application 61/601,244 filed February 21, 2012.

### FIELD OF THE INVENTION

This invention relates to methods and kits for signal amplification of bioassays.

### BACKGROUND

Immunoassays are important tools for both research and diagnostic assays. Thus, there is a need to improve the sensitivity of such assays.

WO 2004/009848 discloses microparticle based amplification wherein microparticles are attached to signalling molecules and analyte binding molecules.

WO 03/104424 discloses molecular conjugates which amplify signaling through analyte-specific binding reagents with a detectable label.

Galikowska et al., Eur J Clin Microbiol Infect Dis 30(9):1067-1073(2011) discloses that bacteria can be detected by bacteriophages using the ELISA-based detection assay.

Gutierrez et al., J Food Protection 60(1):23-27 (1997) discloses that bacteria can be detected by monoclonal antibodies in an ELISA-based detection assay.

### SUMMARY

Embodiments of the present disclosure comprise amplification methods for the detection of an analyte of interest and kits for performing such assays. The present disclosure may be embodied in a variety of ways.

In one embodiment, the invention, as defined in the claims, comprises a method for signal amplification comprising: adding to the analyte of interest a detection support comprising a solid support comprising a plurality of molecules of a binding agent that recognizes and binds to the analyte of interest; and detecting at least some of the plurality of binding agent molecules on the detection support. In an embodiment, the method may further comprise adding a capture support, the capture support comprising at least one capture support binding agent that recognizes and binds to the analyte of interest so as to immobilize the analyte of interest on the capture support. The method may, in certain embodiments also comprise adding a binding agent that can specifically recognize and bind to at least some of the plurality binding agent molecules on the detection support. The binding agent that can specifically recognize and bind to at least some of the plurality binding agent molecules on the detection support may, in some embodiments, comprise a detectable moiety.

In yet other embodiments, the invention comprises kits for performing the methods disclosed herein.

### BRIEF DESCRIPTION OF THE FIGURES

The present invention may be better understood by referring to the following non-limiting figures.
Figure 1, panels A and B, illustrates an assay in accordance with an embodiment of the present invention, where panel A depicts an embodiment of an amplified immunoassay of the invention, and panel B depicts a standard sandwich immunoassay of the prior art.
Figure 2, panels A-E, illustrates a variety of detection methods for proteins of interest in accordance with alternate embodiments of the invention.

### DETAILED DESCRIPTION

### Definitions

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well-known and commonly used in the art. Known methods and techniques are generally performed according to conventional methods well known in the art and as described in various general and more specific references that are discussed throughout the present specification unless otherwise indicated. Enzymatic reactions and purification techniques are performed according to manufacturer's specifications, as commonly accomplished in the art or as described herein. The nomenclatures used in connection with the laboratory procedures and techniques described herein are those well-known and commonly used in the art.

The following terms, unless otherwise indicated, shall be understood to have the following meanings:
As used herein, the terms "a", "an", and "the" can refer to one or more unless specifically noted otherwise.

The use of the term "or" is used to mean "and/or" unless explicitly indicated to refer to alternatives only or the alternatives are mutually exclusive, although the disclosure supports a definition that refers to only alternatives and "and/or." As used herein "another" can mean at least a second or more.

Throughout this application, the term "about" is used to indicate that a value includes the inherent variation of error for the device, the method being employed to determine the value, or the variation that exists among samples.

The term "solid support" or "support" means a structure that provides a substrate onto which biomolecules may be bound. For example, a solid support may be an assay well (i.e., such as a microtiter plate), or the solid support may be a location on an array, or a mobile support, such as a bead.

The term "antibody" includes monoclonal antibodies, polyclonal antibodies, synthetic antibodies and chimeric antibodies, e.g., generated by combinatorial mutagenesis and phage display. The term "antibody" also includes mimetics or peptidomimetics of antibodies. Peptidomimetics are compounds based on, or derived from, peptides and proteins. The peptidomimetics of the present invention typically can be obtained by structural modification of a known peptide sequence using unnatural amino acids, conformational restraints, isosteric replacement, and the like.

The term "binding agent" refers to a molecule that can specifically and selectively bind to a second (i.e., different) molecule of interest. The interaction may be noncovalent, for example, as a result of hydrogen-bonding, van der Waals interactions, or electrostatic or hydrophobic interactions, or it may be covalent. The term "soluble binding agent" refers to a binding agent that is not associated with (i.e., covalently or non-covalently bound) to a solid support.

As used herein, an "analyte" refers to a molecule or compound that is being measured. In some embodiments, the analyte interacts with a binding agent. As described herein, the term "analyte" may refer to a protein or peptide of interest. An analyte may be an agonist, an antagonist, or a modulator. Or, an analyte may not have a biological effect. Analytes may include small molecules, sugars, oligosaccharides, lipids, peptides, peptidomimetics, organic compounds and the like.

The term "detectable moiety" or "detectable biomolecule" or "reporter" refers to a molecule that can be measured in a quantitative assay. For example, a detectable moiety may comprise an enzyme that may be used to convert a substrate to a product that can be measured (e.g., a visible product). Or, a detectable moiety may be a radioisotope that can be quantified. Or, a detectable moiety may be a fluorophore. Or, a detectable moiety may be a luminescent molecule. Or, other detectable molecules may be used.

As used herein, the term "equivalence zone" indicates the region in a precipitin reaction in which the concentration of antigen and antibody leads to maximal precipitation. Thus, if either antigen or antibody is in excess, precipitation does not occur.

### Methods of Signal Amplification

In certain aspects, the present invention utilizes the high specificity of biomolecules that have been coupled to a solid support to further amplify a signal as a means to detect low levels of the analytes (e.g., a protein of interest) present in a sample. In certain embodiments, for immunochemical (for example, ELISA or RIA) or immunofluorescence assays in which a protein to be identified is bound directly or through capture antibodies to a passivated surface, signal amplification of at least 10,000-fold may be achieved.

Thus, in certain embodiments, the present invention comprises a method for signal amplification comprising adding to the analyte of interest a detection support, the detection support comprising a solid support comprising a plurality of molecules of a binding agent that recognizes and binds to the analyte of interest; and detecting at least some of the plurality of binding agent molecules on the detection support. In certain embodiments, the method may further comprise adding a capture support, the capture support comprising at least one capture support binding agent that recognizes and binds to the analyte of interest so as to immobilize the analyte of interest on the capture support. In an embodiment, the analyte of interest is bound to the capture support prior to interacting with the detection support. Or, the analyte of interest may be bound to the capture support after interacting with the detection support. The method further comprises adding a binding agent that can specifically recognize and bind to at least some of the plurality binding agent molecules on the detection support. In an embodiment, the binding agent that can specifically recognize and bind to at least some of the plurality binding agent molecules on the detection support is a soluble binding agent.

In an embodiment, the capture solid support may be an assay well (i.e., such as a microtiter plate). Or, the capture solid support may be a location on an array, or a mobile support, such as a bead. In an embodiment, the detection support is a mobile support such as bead. In certain embodiments, the analyte of interest may be in solution. Or, the analyte of interest may be a protein inside of a microorganism and/or tissue such that upon fixation, macromolecules in the cell function as a capture support. For example, in an embodiment, the immunoassay amplification methods may be used for *in situ* detection of proteins.

In one embodiment, the detection support comprising a plurality of molecules of a binding agent that specifically recognizes the analyte of interest are added in excess to a sample comprising the analyte of interest. Also in an embodiment, the detection support comprises a plurality of molecules that comprise a detectable moiety. Alternatively, where a binding agent that can specifically recognize and bind to at least some of the plurality binding agent molecules on the detection support is used, the soluble binding agent may comprise a detectable moiety.
agent molecules on the detection support is used, the soluble binding agent may comprise a detectable moiety.

A variety of binding agents may be used in the methods of the invention. For example, the plurality of binding agents attached to the detection support may be either an antibody or an antibody fragment that recognizes the analyte of interest. Additionally and/or alternatively, the binding agent attached to the capture support may be an antibody or an antibody fragment that recognizes the analyte of interest. Additionally and/or alternatively, the binding agent that can specifically recognize and bind to at least some of the plurality binding agent molecules on the detection support may be an antibody or an antibody fragment. Or, the binding agent on any of the capture or detection supports, or the binding agent that can specifically recognize and bind to at least some of the plurality binding agent molecules on the detection support may comprise a protein that binds a non-protein target (i.e., such as a protein that specifically binds to a small molecule analyte of interest, or a receptor that binds to a protein).

In an embodiment, the binding agent that can specifically recognize and bind to at least some of the plurality binding agent molecules on the detection support does not recognize the capture binding agent used to bind the analyte of interest to the capture solid support.

The use of a detection support comprising a plurality of binding agents that recognize the analyte of interest provides amplification of the signal. In alternate embodiments, the detection support comprises more than 1,000, or more than 10,000, or more than 100,000, or more than 500,000, or more than 1,000,000 binding agent molecules specific for the analyte of interest. Thus, in alternate embodiments, the method of the invention provides an amplification of 1,000, or more than 10,000, or more than 100,000, or more than 500,000, or more than 1,000,000 times the signal seen in a standard, unamplified immunoassay that does not comprise a detection support comprising a plurality of detection binding agents. Thus, in alternate embodiments, the methods of the invention provides an amplification that ranges from 1,000 to 1,000,000,000, or from 1,000 to 100,000,000, or from 5,000 to 10,000,000, or from 10,000 to 1,000,000, or from 10,000 to 500,000, or from 50,000 to 500,000 times the signal seen in a standard, unamplified immunoassay that does not comprise a detection support comprising a plurality of detection binding agents. Or ranges within these ranges may be achieved.

In certain embodiments the analyte of interest is a protein and the binding agents are antibodies. Thus, in certain embodiments the present invention comprises a method for signal amplification of the detection of proteins by an immunoassay.

The use of the detection support and/or the capture support may comprise a variety of formats.

For example, in some embodiments, the analyte of interest may first be bound to the capture support and then allowed to interact with the detection support. Thus, the method may comprise the steps of attaching a plurality of binding agents that can specifically bind to a protein of interest to a capture support. The method may further comprise adding the analyte of interest to the capture support. Next the method may comprise adding to the analyte of interest that is bound to the capture support a detection support comprising a plurality of detection binding agents specific for the analyte of interest, such that the detection of the plurality of detection binding agents provides amplification of the signal.

Also, in certain embodiments, the method may comprise adding a third binding agent that can specifically recognize and bind to the plurality of detection binding agents. In an embodiment, the binding agent that can specifically recognize and bind to the plurality of detection binding agents is a soluble binding agent. The third binding agent may comprise a detectable moiety. Thus, in some embodiments, performing the steps of the method generates a complex comprised of the capture support: capture binding agent: analyte of interest: detection binding agent: detection support: soluble binding agent: detectable moiety.

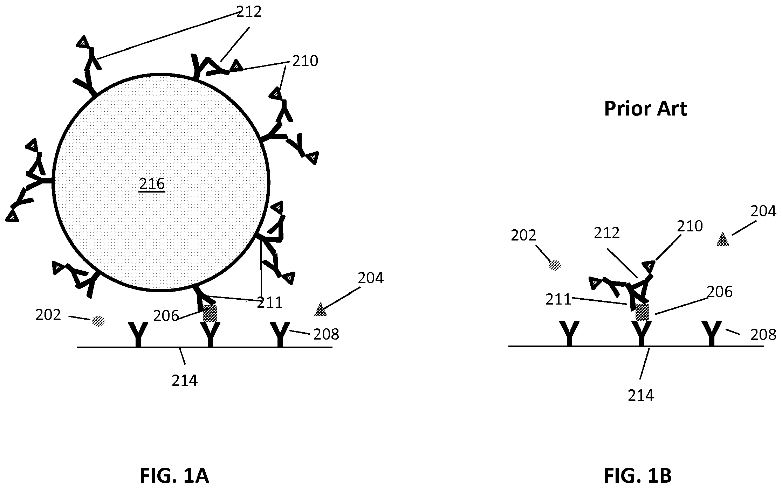

Figure 1A depicts an embodiment of an amplified immunoassay of the invention according to the format above. For example, and as depicted in Figure 1A, the method may comprise the steps of fixing a plurality of binding agents 208 to a capture solid support 214 such that the binding agents can specifically recognize and capture a protein of interest 206. In an embodiment, the protein may be isolated from a cell membrane or released from a cell of interest and then added to the solid phase. In an embodiment, the capture binding agent attached to the capture solid support is a primary antibody, or an antibody fragment that recognizes the protein. For example, in one embodiment, the capture support 214 may be a microtiter well coated with antibodies 208 to the protein of interest 206 (e.g., rabbit antibody). Or, other binding agents, e.g., receptors that recognize specific ligands, nucleic acid molecules that can hybridize to complementary sequences, polysaccharides, lipids, lipopolysaccharides, teichoic acids, or lipoteichoic acids that recognize specific ligands, and the like, may be used. The immobilized antibodies 208 may specifically recognize the analyte of interest 206, whereas other proteins or biomolecules, 202, 204 are not bound.

Next a detection support 216 (e.g., a bead) coated with a plurality of molecules of a detection binding agent 211 that also recognizes the analyte of interest 206 may be added. In an embodiment, the detection support may comprise thousands, to tens of thousands, to hundreds of thousands of the detection binding agent. In an embodiment, the detection binding agent is an antibody. For example, the capture binding agent may be a rabbit antibody that recognizes a protein of interest, whereas the detection binding agent may be a guinea pig primary antibody that also recognizes the protein of interest. In this way, the protein of interest 206 links the detection support 216 comprising the plurality of detection binding agent molecules 211 that recognize the protein of interest 206 bound to the capture binding agent 208 immobilized on the capture solid support 214.

Next, a soluble binding agent (e.g., a secondary antibody such as anti-guinea pig antibody) 212 that recognizes the detection binding agent 211 may be added. In an embodiment, the soluble binding agent 212 is labeled with a detectable moiety 210 (i.e., a reporter). For example, the soluble binding agent may be labeled with a fluorescent moiety (e.g., QDOTS®) or an enzyme (e.g., horseradish peroxidase). Or, in an embodiment, the detection binding agents on the second support may be labeled with such detectable moieties. Thus a very large amplification of the signal may originate from a single protein molecule 206.

Thus, as shown in Figure 1A, as the protein 206 can recognize both of the primary antibodies (i.e., a guinea pig antibody 211 on the bead 216 and a rabbit antibody 208 on the capture support 214), it can facilitate formation of a sandwich complex between at least one of the beads 216 and the antibody bound 208 to the solid surface 214. Then, after washing away of any unbound beads 216, the beads bound to the protein 206 of interest can be detected using a secondary antibody 212 that recognizes the primary antibody 211 bound to the bead. In an embodiment, the secondary antibody is attached to a detectable moiety 210 such as an enzyme, a fluorophore, QDOTS®, or the like.

Thus, the method may comprise the steps of fixing a plurality of binding agents to a solid support ("capture support"), such that the binding agents can specifically recognize and capture an analyte of interest. Next, the method may comprise adding a mobile second support (e.g., a bead or beads) coated with tens of thousands of a second binding agent ("detection support") that can specifically bind to at least one molecule immobilized on the first support. The method may further include the step of adding a third binding agent-reporter complex (e.g., a soluble binding agent coupled to a detectable moiety) that specifically binds to at least some of the plurality of the second binding agent on the second support. Next the method may comprise the step of detecting the complex comprised of the capture support: capture binding agent: analyte of interest: detection binding agent: detection support: soluble binding agent: detectable moiety. As illustrated in Figure 1A, due to the large number of binding agent molecules 211 attached to the detection support 216, which can bind the soluble binding agent 212 with reporter molecules 210, amplification of the signal (i.e., protein 206 bound to the binding agent 208 on the capture support 214) is increased dramatically.

Figure 1B provides an illustration of an unamplified immunoassay of the prior art for comparison. Thus, the immunoassay of Figure 1B comprises the formation of a sandwich comprising a capture support 214: first primary antibody 208: analyte of interest 206: second primary antibody 211: secondary antibody 212: detectable moiety 210.

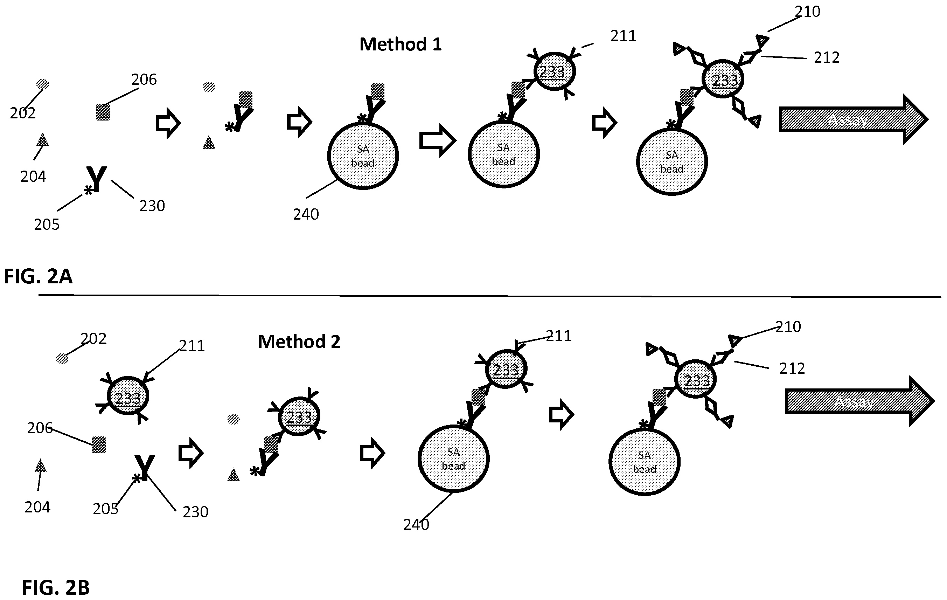

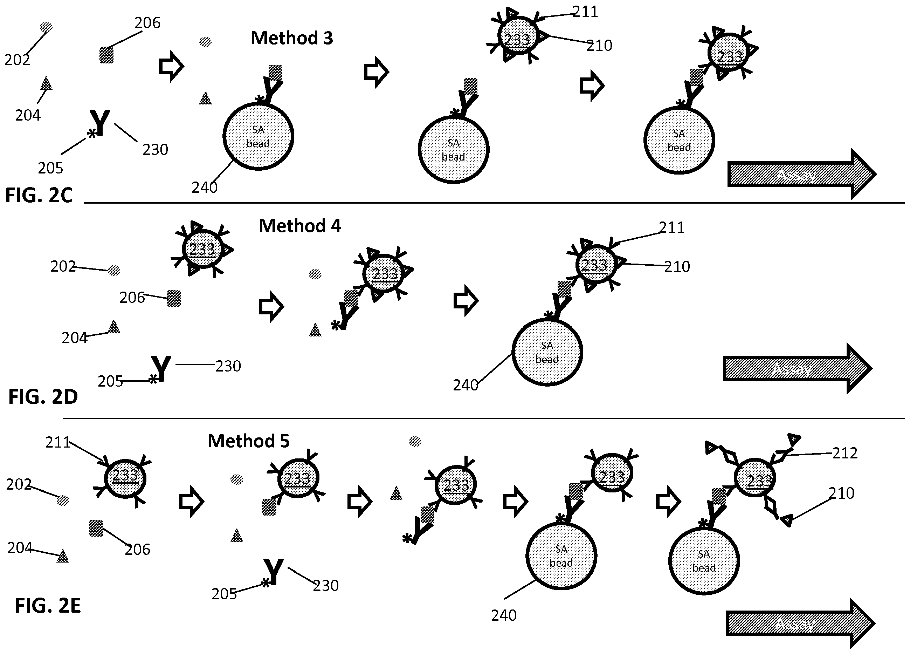

Figure 2 illustrates several alternate embodiments of amplified immunoassays that may be used to detect analytes of interest where the order that various components are added to the assay is varied.

As shown in Figure 2A (Method 1), in some embodiments, the analyte of interest may be bound to a capture binding agent 230, and then the capture binding agent attached to the capture support 240. Then, the detection support 233 comprising a plurality of detection binding agents 211 may be added. Finally, a soluble binding agent 212 comprising a detectable moiety 210 and that recognizes the detection binding agents 211 is added.

For example in one embodiment, molecules of a primary antibodies 230 (e.g., rabbit) specific for an analyte of interest 206 (e.g., a protein of interest) and labeled with biotin 205 are added so as to bind to the analyte molecules 206 in solution. The biotinylated antibodies 230 may specifically recognize the analyte of interest 206, whereas other proteins or biomolecules, 202, 204 are not bound. At this point, a magnetic streptavidin-coated "capture" bead (or beads) 240 (e.g., about one micrometer in diameter) may be added to bind the biotinylated antibody-protein complexes quantitatively. In an embodiment, the method may include blocking the bead-protein complexes by the addition of biotin and bovine serum albumin (BSA).

At this point, a "detection" bead or beads 233 coated with thousands, to tens of thousands, to hundreds of thousands molecules of a second primary antibody 211 that recognizes the protein of interest 206, but that is produced in a different species (e.g., guinea pig) than the biotinylated capture antibodies 230 are added to bind to open, unoccupied epitopes on the analyte 206. The amount of detection antibody 211 may be detected using a secondary antibody 212 that recognizes the second primary antibody 211 (such as anti-guinea pig antibody for a guinea pig primary antibody). In an embodiment, the secondary antibody 212 is labeled with a detectable moiety 210. For example, the secondary antibody may be labeled with a fluorescent moiety (e.g., QDOTS®) or an enzyme (e.g., horseradish peroxidase). Thus a very large amplification of the signal can originate from a single protein molecule.

Figure 2B (Method 2) illustrates an alternate form of the amplified assay of Method 1, but where the detection support or supports 233 comprising detection binding agent molecules 211 are added to the assay well with the capture binding agent 230 prior to the addition of the capture support 240 that recognizes the capture binding agent 230. Once the complex(es) of capture support: capture binding agent: analyte of interest: detection binding agent: detection support is formed, the molecules of a soluble binding agent 212 labeled with a detectable moiety 210 may be added.

Figure 2C (Method 3) illustrates an alternate form of the assay of Method 1, but where the detection support or supports 233 comprising a plurality of detection binding agents 211 also comprise a plurality of molecules of a detectable moiety 210 attached to (e.g., by covalent attachment or coating the surface) the surface of the detection supports rather than being bound to a separate soluble binding agent. In an embodiment, this method may allow for the use of fewer detection binding agents if the detection support comprises a plurality of detectable moieties. For example, the ratio of detectable moieties: detection binding agents on the detection support may be 1:1, or 5:1, or 10:1, or 100:1, or 500:1, or 1000:1, or 10,000:1 or greater. The detectable moiety may comprise a fluorescent moiety (e.g., QDOTS®) or an enzyme (e.g., horseradish peroxidase). Also, it can be seen that in this embodiment, there is no need to use a binding agent that can specifically recognize and bind to at least some of the plurality binding agent molecules on the detection support (e.g., soluble antibody 212).

Figure 2D (Method 4) illustrates an alternate form of the assay of Method 3, but where the detection support or supports 233 coated with detection binding agents 211 and a plurality of detectable moieties 210 are added prior to the addition of the capture support 240. In some embodiments, the detection support(s) 233 coated with detection binding agents 211 and a plurality of detectable moieties 210 and the capture binding agent 230 are added simultaneously. In an embodiment, this may promote formation of a complex between the analyte of interest 206 and the detection support 233 and the capture binding agent 230.

Figure 2E (Method 5) illustrates an alternate form of Method 2, but where the detection support(s) 233 is added prior to the addition of the capture binding agent 230 and the capture support 240. In an embodiment, this may promote formation of a complex between the analyte of interest 206 and the detection support 233.

It can be important that the binding agent that can specifically recognize and bind to at least some of the plurality binding agent molecules on the detection support and/or the detection binding agent do not bind to the capture support or capture support binding agent, as such binding could lead to background. Thus, in certain embodiments, the step of detection comprises addition of a soluble secondary antibody that recognizes the detection primary antibody agent on the detection bead, but wherein the soluble secondary antibody does not recognize the capture binding agent (e.g., first primary antibody) used to bind the protein of interest to the capture support.

In certain embodiments, the capture and/or detection solid supports may be treated with a passivating agent. For example, in certain embodiments the analyte of interest may be captured on a passivated surface (i.e., a surface that has been treated to reduce non-specific binding). One such passivating agent is BSA. Additionally and/or alternatively, where the binding agent used is an antibody, the capture and/or detection solid supports may be coated with protein A, protein G, protein A/G, protein L, or another agent that binds with high affinity to the binding agent antibody. These proteins bind the Fc domain of antibodies and thus can orient the binding of antibodies that recognize the protein or proteins of interest.

The detection and/or capture supports may be polystyrene beads or beads made of a similar or other material, such that the beads that can be coated with proteins, but do not react with other components in the assay. In certain embodiments the beads are sufficiently large such that a plurality of the antibody molecules may be attached to the bead. For example, the beads may range in size from about 0.1 to 50, or 0.2 to 40, or 0.3 to 30, or 1 to 20, or 1 to 15 or about 1 to 3 µm in diameter. The size of the beads may depend upon the size of the assay to be performed. For an assay performed in a microtiter well, beads of about 1 micrometer (µm) may be used.

In alternate embodiments, the detection support may comprise a plurality of detection binding agents. For example, in alternate embodiments, the number of binding agents on the detection support may be greater than 100, or greater than 500, or greater than 1,000, or greater than 5,000, or greater than 10,000, or greater than 20,000, or greater than 50,000, or greater than 100,000, or greater than 500,000, or greater than 1,000,000 molecules of the detection binding agent. For example, in an embodiment, the detection support may comprise a bead that is coated with tens or hundreds of thousands of antibody molecules. In alternate embodiments, there may be about 10,000 to 10,000,000 or about 50,000 to 500,000, or about 100,000 to 1,000,000 binding agent molecules (e.g., a primary antibody) for a detection support bead that is about 15 µm in diameter. For detection support beads of different sizes, corresponding surface coverage can be used. Or ranges within these ranges may be achieved.

As described above, a binding agent complexed with an enzyme or fluorescent material (QDOTS® or other fluorescent label) may be added to bind to the large number of antibodies used as detection binding agents on a detection support. Immunochemical (for example, ELISA) assay or excitation and visualization of fluorescent material in an appropriate emission imaging system may be used to quantify the protein. In an embodiment, the key to the large signal amplification factor is the large surface area of the detection support that can accommodate binding of detection binding agents. For example, for antibodies, about >10,000 and >100,000 molecules may be used to coat 1 µm and 2.8 µm beads, respectively.

The area occupied by the molecules of the analyte of interest on the capture support should satisfy accessibility to the surface of the detection support. For example, a two-dimensional, confluent array of 1,000,000 beads functioning as a capture supports and having a size of 1µm may occupy about 1 mm². In an embodiment, serial dilutions of a sample containing 10⁶ molecules of the analyte of interest may be applied to a fixed (e.g., microtiter well) capture support so as to yield separate locations for each sample application. For a mobile capture support (e.g., a bead) the number of detection supports of a particular size that can bind can be limited by steric effects. For either fixed or mobile supports, individual molecules of the analyte of interest that can bind to a 1 µm or 2.8 µm bead coated with >10⁴ or >10⁵ primary antibodies, respectively, may provide amplification in this range by ELISA, RIA or immunofluorescence assay upon binding of labeled secondary antibodies.

For example, in one embodiment, the invention may comprise a method of amplifying the signal in indirect immunochemical or immunofluorescent assays by ~10,000-fold comprising: binding of polystyrene detection beads (typically one micrometer in diameter) coated with protein A/G and >10,000 antibody molecules specific for the protein to be identified to the protein molecules immobilized (either directly or through specific capture antibodies) with appropriate spacing on a passivated surface or immobilized on a capture bead; removal of unbound bead-antibody complexes with washes; binding of secondary antibodies complexed with, for example, an enzyme or fluorescent material (QDOTS®) or other fluorescent label); and quantifying the protein by, for example, ELISA or by excitation and visualization of the fluorescent material in an appropriate emission imaging system.

Additionally and/or alternatively, the analyte of interest may be in solution, and the assay may comprise having the analyte link a plurality of detection supports together as a means of detecting the analyte. For example, a protein in solution may be used to link a plurality of detection beads (e.g., about 15 to 20 µm in diameter), the detection beads each having a plurality of antibodies, thereby promoting clumping of the beads via binding to antibodies on two beads. This may be particularly effective where polyclonal antiserum is used, as such antisera will allow for the antibodies to recognize different epitopes on the protein of interest. Serial dilutions of the protein may be tested for maximum bead agglutination in order to determine the optimal concentrations of antibody-beads and protein, i.e., the equivalence zone. A prozone effect of antibody excess is unlikely as the antibodies are immobilized on the beads.

In another embodiment, where the analyte of interest is within a microorganism or a tissue sample, cells in a sample can be concentrated onto the surface of a filter of appropriate pore size (e.g., typically 0.45 µm spin filter for bacteria). Once concentrated, they can be lysed in a small volume of buffer as described herein to release molecules of the analyte of interest. The cell lysate containing the analyte of interest can be transferred from the filter surface to a glass slide or to the well of a microtiter plate. At this point, the analyte of interest (e.g., a protein) can be identified by formation of a lattice following the addition of ~ 300 to ∼1,000 large (typically 15 to 20 µm in diameter) polystyrene beads coated with protein G, protein A or proteins A/G and specific antibodies. Bead clumping can be visualized directly or with the aid of magnification at 50 to 100 times. Again, immobilization of the antibodies on the beads can obviate prozone effects; however, serial dilutions of the protein mixed with a constant number of bead-antibody complexes may be required to observe bead clumping because of possible antigen excess. Bead-protein G-antibody complexes aggregated (clumped) with a known amount of the analyte of interest may serve as a positive control, and unaggregated bead-protein G-antibody complexes in buffer without any of the analyte of interest (e.g., a lysate from a known negative control) may serve as a negative control.

### Kits For Signal Amplification

In alternate embodiments, the present invention may comprise kits to perform the methods of the invention. In an embodiment, the invention may comprise a kit for assaying an analyte of interest comprising a detection support, the detection support comprising a solid support comprising a plurality of binding agent molecules that can recognize and bind to the analyte of interest. In an embodiment, the kit may comprise a capture support, the capture support comprising at least one capture support binding agent that recognizes and binds to the analyte of interest. The kit may, in some embodiments, further comprise a binding agent that can specifically recognize and bind to the plurality of binding agent molecules on the detection support. In an embodiment, the binding agent that can specifically recognize and bind to the plurality of binding agent molecules on the detection support is a soluble binding agent.

The detection and/or capture supports may comprise a variety of formats. In an embodiment, the capture solid support may be an assay well (i.e., such as a microtiter plate). Or, the capture solid support may be a location on an array, or a mobile support, such as a bead. In an embodiment, the detection support is a mobile support such as bead.

A variety of binding agents may be used in the kits of the invention. For example, the plurality of binding agent molecules attached to the detection support may be either molecules of an antibody or an antibody fragment that recognizes the analyte of interest. Additionally and/or alternatively, the binding agent attached to the capture support may be an antibody or an antibody fragment that recognizes the analyte of interest. Additionally and/or alternatively, the binding agent that can specifically recognize and bind to the plurality of binding agent molecules on the detection support may be an antibody or an antibody fragment. In an embodiment, the binding agent that can specifically recognize and bind to the plurality of binding agent molecules on the detection support does not recognize the capture binding agent used to bind the analyte of interest to the capture solid support. Or, the binding agent on any of the capture or detection supports, or the binding agent that can specifically recognize and bind to the plurality of binding agent molecules on the detection support may comprise a protein that binds a non-protein target (i.e., such as a protein that specifically binds to a small molecule analyte of interest, or a receptor that binds to a protein).

The detection support may comprise a plurality of molecules of a detectable moiety. Additionally and/or alternatively, the binding agent that can specifically recognize and bind to the plurality of binding agent molecules on the detection support may comprise a detectable moiety.

The use of a detection support comprising a plurality of binding agents that recognize the analyte of interest provides amplification of the signal. In alternate embodiments, the detection support may comprise a plurality of detection binding agents. For example, in alternate embodiments, the number of binding agents on the detection support may be greater than 100, or greater than 500, or greater than 1,000, or greater than 5,000, or greater than 10,000, or greater than 20,000, or greater than 50,000, or greater than 100,000, or greater than 500,000, or greater than 1,000,000 molecules of the detection binding agent. For example, in an embodiment, the detection support may comprise a bead that is coated with tens or hundreds of thousands of antibody molecules. In alternate embodiments, there may be about 10,000 to 10,000,000 or about 50,000 to 500,000, or about 100,000 to 1,000,000 binding agent molecules (e.g., a primary antibody) for a detection support bead that is about 15 µm in diameter. For detection support beads of different sizes, corresponding surface coverage can be used. Or ranges within these ranges may be achieved.

Thus, in alternate embodiments, the method of the invention provides an amplification of 1,000, or more than 10,000, or more than 100,000, or more than 500,000, or more than 1,000,000 times the signal seen in a standard, unamplified immunoassay that does not comprise a detection support comprising a plurality of detection binding agents. For example, in alternate embodiments, the kits of the invention provides an amplification that ranges from 1,000 to 1,000,000,000, or from 1,000 to 100,000,000, or from 5,000 to 10,000,000, or from 10,000 to 1,000,000, or from 10,000 to 500,000, or from 50,000 to 500,000 times the signal seen in a standard, unamplified immunoassay that does not comprise a detection support comprising a plurality of detection binding agents. Or ranges within these ranges may be achieved.

Thus, in certain embodiments, the kit may comprise reagents for a bead-based immunoassay. Thus, in one embodiment, the detection support may comprise a bead coated with molecules of an antibody (or antibody fragment) that recognizes a protein of interest. The bead may in certain embodiments be a polystyrene bead coated with proteins G, protein A, or proteins A/G, which are available commercially (e.g., Life Technologies/Invitrogen, Carlsbad CA) in a wide variety of sizes. Other beads that may be useful include magnetic silica beads coated with these same proteins (MagSi beads from AmsBio, Lake Forest, CA).

In alternate embodiments, there may be about 10,000 to 10,000,000 or about 50,000 to 1,000,000, or about 100,000 to 500,000 binding agent molecules (e.g., a primary antibody) for a detection support bead that is about 15 µm in diameter. For detection support beads of different sizes, corresponding surface coverage can be used. In certain embodiments the detection support beads are sufficiently large such that a plurality of the binding agent molecules may be attached to the bead. For example, the beads may range in size from about 0.1 to 50, or 0.2 to 40, or 0.5 to 30, or 1 to 20, or 1 to 15 or about 1 to 3 µm in diameter. Where the binding agent to be attached to the bead is an antibody, beads commercially available pre-coated with protein G, protein A, or proteins A/G may be used as these proteins bind the Fc domain of antibodies, orienting the antibodies such that the Fab domains are free to bind epitopes of antigens.

The kits for a bead-based immunoassay may also comprise additional primary antibodies for the protein of interest, where such antibodies are from a second species. These antibodies have particular utility in indirect assays as described herein as they will accommodate specific binding of secondary anti-antibody-reporter molecule complexes. Moreover, these secondary antibody-reporter complexes will not bind to the primary antibodies from the first species that fix the target protein to the solid surface or to the protein A/G beads, obviating false positive reactions. Additionally, the secondary antibody-reporter complexes will not bind to the surface of protein A/G beads, thereby obviating false positive reactions.

The kits may also comprise soluble secondary antibodies that may be labeled with a detectable marker or a binding agent that can complex with a detectable marker. For example, in certain embodiments, a kit of the invention may comprise a secondary antibody that is bound to a fluorophore. In an embodiment, the fluorophore may comprise a QDOT®. For example, in one embodiment, anti-antibody-QDOT® conjugates recognize the plurality of bead-bound primary antibodies.

Substrates to be used in the kits of the present invention include, but are not limited to, polystyrene beads (Spherotech, Libertyville, Ill.), magnetic beads (Invitrogen; AmsBio), latex coatings, a membrane filter, a fiber filter, a free fiber or a porous solid substrate. Methods for the use of magnetic beads can be found, for example, with the package insert of Dynabeads Protein G Prod. No. 10003D, in Kala et al., Analytical Biochemistry 254:263-266 (1997) and in Dutton, Genetic Engineering News, Volume 22, Number 13, July 2002.

A wide spectrum of particles, particularly magnetic and polystyrene beads, is commercially available in a wide range of sizes. For certain embodiments, a preferred set of particles has an average particle size (i.e., the largest dimension of the particles) of about one micrometer (i.e., micron). For certain embodiments, a preferred set of particles has an average particle size (i.e., the largest dimension of the particles) of no less than 10 micrometers (i.e., microns). Exemplary commercially available beads are protein G-, protein A-, and proteins A/G- coated polystyrene beads and streptavidin-coated polystyrene beads that are available from Invitrogen, Carlsbad, CA or from Spherotech, Libertyville, Ill. Other suppliers of polystyrene and magnetic beads include Thermo Scientific Pierce, Millipore, Polyscience and New England Biolabs. AmsBio, Lake Forest, IL is a supplier of magnetic silica beads, which are available with all of the protein coatings given above. Invitrogen also supplies epoxy surface magnetic beads, which bind antibodies covalently. The beads may be
polystyrene, silica, glass, or other, plane or derivatized for attachment to specific chemical groups.

### EXAMPLES

Embodiments of the present invention may also be characterized by the following non-limiting examples. Any examples not falling within the scope of the claim are provided for comparative purposes only.

### Example 1: Detection of Proteins with Bead-Based Signal Amplification

A protein or protein subunit of interest may be captured by antibodies specific to the protein of interest (e.g., biotinylated rabbit primary antibodies) immobilized on a passivated surface such as Neutravidin to minimize false positives (e.g., see Jain et al., Nature, 2011, 473:484-488). Next, in a sandwich assay, 1 µm or 2.8 µm polystyrene beads coated with protein A/G and another type of antibodies specific to the protein (typically >10⁴ and >10⁵ for 1 µm and 2.8 µm beads, respectively) produced in a second species (e.g., guinea pigs) are added to bind to the immobilized target protein subunits. Then secondary antibodies or antibody fragments (e.g., anti-guinea pig antibodies) that are conjugated (e.g., covalently bound) to an enzyme, radioactive tag, or fluorescent material such as QDOTS® are added to bind to the plurality of bead-bound primary antibodies specific for the analyte of interest. Unbound protein molecules or antibodies are removed by washes (2 times) between successive additions.

ELISA, RIA or immunofluorescence assays can be used to detect and quantify the protein or protein subunit of interest. To assay fluorescence, the spots are excited with UV light and visualized with an appropriate emission filter in a hyperspectral imaging system with a camera.

### Example 2: Capture of Ribosomal Proteins Using Various Formats of Bead

**Amplification** Experiments were performed to compare the various assay formats depicted in Figure 2, panel B (i.e., Method 2). Essentially, *E. coli* lysates (500,000 cells) were prepared by lysing bacterial cells, and dissociating ribosomes therein to protein constituents in 6 M (10,000 cells/µl) guanidine thiocyanate. The guanidine thiocyanate concentration was then reduced to 0.12 or 0.6 M by dilution into phosphate buffer (e.g., dilution of 90,000 cells into 450 µl). Ribosomal proteins were then captured using one of the capture detection methods shown in Figure 2 (Table 1). Results are show for initial input cells and for estimated ribosomal protein from the cells; 100 *E. coli* cells have about 2.7 pg of ribosomal protein.

| Method | No. *E. coli* cells | Estimated Ribosomal Protein | Signal over 0 cell control |
|---|---|---|---|
| Method 2 | 2,000 | 54 pg | 6.17 |
| | 10,000 | 270 pg | 3.16 |
| Method 5 (1)* | 4,000 | 108 pg | 9.3 |
| Method 5 (2) | 500 | 14 pg | 1.8 |
| | 2,000 | 54 pg | 2.2 |

| | | | |
|---|---|---|---|
| *Experiments 1 and 2. | | | |

Additional experiments were performed using the method depicted in panel 2E. This method utilizes addition of the bead-Ab2 (carboxy beads and rabbit anti-ribosome antibodies) to the lysate. This allows the ribosomal proteins to bind to the bead, similar to a regular sandwich ELISA where the 'capture' antibodies are immobilized on a surface. The Ab1-biotin (guinea pig) was then added directly to the mixture without any wash steps. The bead-Ab2-protein-Ab1 complexes are captured using streptavidin (SA) beads and are detected using secondary antibodies to Ab2. It was found that this approach worked when rabbit Ab-carboxy beads (Ab2-bead) and biotin, guinea pig antibodies (Ab1) were used.

Thus, in this experiment, lysate from 2,500/5,000/10,000/20,000 cells were incubated with about 1.3 x 10⁸ carboxy beads (0.1 µm) with rabbit anti-ribosome antibody (anti-ribo Ab). Then, 10 ng (4 x 10¹⁰ IgG molecules) biotinylated guinea pig, anti-ribosome antibodies were added and incubated further. The bead-Ab-ribo protein-Ab complexes were captured with about 4 x 10⁷ C1 SA Dynabeads. Detection was performed with 10 ng anti-rabbit antibody labeled with horse radish peroxidase (HRP). One-fifth of the sample was loaded onto a microplate for detection with Sirius HRP substrate in a luminometer. It can be seen that the signal was detected for as little as 500 cells corresponding to about 14 pg of ribosomal protein.

## Claims

1. A method for signal amplification comprising:
adding to the analyte of interest a detection support comprising a solid support comprising a plurality of molecules of a binding agent that recognizes and binds to the analyte of interest; and
detecting at least some of the plurality of binding agent molecules on the detection support by adding a binding agent that can specifically recognize and bind to at least some of the plurality of molecules of the binding agent on the detection support.

2. The method of claim 1, wherein the detection support is a bead; or
wherein the detection support comprises a plurality of molecules of a detectable moiety.

3. The method of any one of claims 1 to 2, further comprising adding a capture support, the capture support comprising at least one capture support binding agent that recognizes and binds to the analyte of interest so as to immobilize the analyte of interest on the capture support; preferably
wherein the analyte of interest is bound to the capture support prior to interacting with the detection support; or
wherein the analyte of interest is bound to the capture support after interacting with the detection support.

4. The method of any one of claims 1 to 3,
wherein the binding agent that can specifically recognize and bind to at least some of the plurality of binding agent molecules on the detection support comprises a detectable moiety,
wherein the binding agent that can specifically recognize and bind to at least some of the plurality of binding agent molecules on the detection support is an antibody or an antibody fragment,
wherein the binding agent that can specifically recognize and bind to at least some of the plurality of binding agent molecules on the detection support does not recognize the capture binding agent used to bind the analyte of interest to the capture solid support, or
wherein the binding agent that can specifically recognize and bind to at least some of the plurality of binding agent molecules on the detection support comprises a soluble binding agent.

5. The method of any one of claims 3 to 4, wherein the capture support is a bead.

6. The method of any one of claims 3 to 4, wherein the binding agent attached to the capture support is an antibody or an antibody fragment that can recognize and bind to the analyte of interest.

7. The method of any one of claims 1 to 6, wherein the plurality of molecules of the binding agent attached to the detection support are antibodies or antibody fragments that recognize and bind to the analyte of interest.

8. The method of any one of claims 1 to 7, wherein the detection support comprises more than 10,000 binding agent molecules specific for the analyte of interest, or wherein the detection support comprises more than 100,000 binding agent molecules specific for the analyte of interest.

9. A kit for assaying an analyte of interest comprising: a detection support, the detection support comprising solid support comprising a plurality of molecules of a binding agent that can recognize and bind to the analyte of interest and a binding agent that can specifically recognize and bind to at least some of the plurality of binding agent molecules on the detection support.

10. The kit of claim 9, further comprising a capture support, the capture support comprising at least one capture support binding agent that recognizes and binds to the analyte of interest.

11. The kit of any one of claim 9 to 10, wherein the detection support is a bead; or wherein the detection support comprises a plurality of molecules of a detectable moiety.

12. The kit of any one of claims 9 to 11, wherein the plurality of binding agent molecules attached to the detection support are antibodies or antibody fragments that can recognize and bind to the analyte of interest.

13. The kit of any one of claims 9 to 10,
wherein the binding agent that can specifically recognize and bind to at least some of the plurality of binding agent molecules on the detection support comprises a soluble binding agent,
wherein the binding agent that can specifically recognize and bind to at least some of the plurality of binding agent molecules on the detection support is an antibody or an antibody fragment that can recognize and bind to the analyte of interest, or
wherein the binding agent that can specifically recognize and bind to at least some of the plurality of binding agent molecules on the detection support comprises a detectable moiety.

14. The kit of any one of claims 10 to 13, wherein the capture support is a bead,
or wherein the capture support binding agent is an antibody or an antibody fragment that can recognize and bind to the analyte of interest.

15. The kit of any one of claims 9 to 14, wherein the detection support comprises more than 1,000 binding agent molecules specific for the analyte of interest;
wherein the detection support comprises more than 10,000 binding agent molecules specific for the analyte of interest; or wherein the detection support comprises more than 100,000 binding agent molecules specific for the analyte of interest.

## Patentansprüche

1. Verfahren zur Signalverstärkung, umfassend:
Hinzufügen zum Analyt von Interesse eines Nachweisträgers, der einen Feststoffträger umfasst, der eine Vielzahl von Molekülen eines Bindemittels umfasst, das den Analyt von Interesse erkennt und bindet; und
Nachweisen mindestens einiger der Vielzahl von Bindemittelmolekülen auf dem Nachweisträger durch Hinzufügen eines Bindemittels, das speziell mindestens einige der Vielzahl von Molekülen des Bindemittels auf dem Nachweisträger erkennen und sich daran binden kann.

2. Verfahren nach Anspruch 1, wobei der Nachweisträger ein Kügelchen ist; oder wobei der Nachweisträger eine Vielzahl von Molekülen eines nachweisbaren Anteils umfasst.

3. Verfahren nach einem der Ansprüche 1 bis 2, ferner das Hinzufügen eines Einfangträgers umfassend, wobei der Einfangträger mindestens ein Einfangträger-Bindemittel umfasst, das den Analyt von Interesse erkennt und bindet, um den Analyt von Interesse auf dem Einfangträger zu immobilisieren; vorzugsweise
wobei der Analyt von Interesse vor dem Wechselwirken mit dem Nachweisträger an den Einfangträger gebunden wird; oder
wobei der Analyt von Interesse nach dem Wechselwirken mit dem Nachweisträger an den Einfangträger gebunden wird.

4. Verfahren nach einem der Ansprüche 1 bis 3,
wobei das Bindemittel, das speziell mindestens einige der Vielzahl von Bindemittelmolekülen erkennen und diese an den Nachweisträger binden kann, einen nachweisbaren Anteil umfasst,
wobei das Bindemittel, das speziell mindestens einige der Vielzahl von Bindemittelmolekülen erkennen und diese an den Nachweisträger binden kann, ein Antikörper oder ein Antikörperfragment ist,
wobei das Bindemittel, das speziell mindestens einige der Vielzahl von Bindemittelmolekülen erkennen und diese an den Nachweisträger binden kann, das Einfangbindemittel nicht erkennt, as verwendet wird, den Analyt von Interesse an den Einfangfeststoffkörper zu binden, oder
wobei das Bindemittel, das speziell mindestens einige der Vielzahl von Bindemittelmolekülen erkennen und diese an den Nachweisträger binden kann, ein lösbares Bindemittel umfasst.

5. Verfahren nach einem der Ansprüche 3 bis 4, wobei der Einfangträger ein Kügelchen ist.

6. Verfahren nach einem der Ansprüche 3 bis 4, wobei das am Einfangträger befestigte Bindemittel ein Antikörper oder ein Antikörperfragment ist, das den Analyt von Interesse erkennen und binden kann.

7. Verfahren nach einem der Ansprüche 1 bis 6, wobei die Vielzahl von Molekülen des an den Nachweisträger befestigten Bindemittels Antikörper oder Antikörperfragmente sind, die den Analyt von Interesse erkennen und sich daran binden.

8. Verfahren nach einem der Ansprüche 1 bis 7, wobei der Nachweisträger mehr als 10.000 Bindemittelmoleküle speziell für den Analyt von Interesse umfasst, oder wobei der Nachweisträger mehr als 100.000 Bindemittelmoleküle speziell für den Analyt von Interesse umfasst.

9. Kit zum Prüfen eines Analyt von Interesse, umfassend: Einen Nachweisträger, wo bei der Nachweisträger einen Feststoffträger umfasst, der eine Vielzahl von Molekülen eines Bindemittels, das einen Analyt von Interesse erkennen und sich an diesen binden kann und ein Bindemittel umfasst, das speziell mindestens einige der Vielzahl von Bindemittelmolekülen auf dem Nachweisträger erkennen und sich an diese binden kann.

10. Bausatz nach Anspruch 9, der ferner einen Einfangträger umfasst, wobei der Einfangträger mindestens ein Einfangträger-Bindemittel umfasst, das den Analyt von Interesse erkennt und sich an diesen bindet.

11. Kit nach einem der Ansprüche 9 bis 10, wobei der Nachweisträger ein Kügelchen ist; oder wobei der Nachweisträger eine Vielzahl von Molekülen eines nachweisbaren Anteils umfasst.

12. Kit nach einem der Ansprüche 9 bis 11, wobei die Vielzahl von Bindemittelmolekülen, die an den Nachweisträger befestigt sind, Antikörper oder Antikörperfragmente sind, die den Analyt von Interesse erkennen und sich an diesen binden können.

13. Kit nach einem der Ansprüche 9 bis 10,
wobei das Bindemittel, das speziell mindestens einige der Vielzahl von Bindemittelmolekülen erkennen und diese an den Nachweisträger binden kann, ein lösbares Bindemittel umfasst,
wobei das Bindemittel, das speziell mindestens einige der Vielzahl von Bindemittelmolekülen erkennen und diese an den Nachweisträger binden kann, ein Antikörper oder ein Antikörperfragment ist, welcher/welches den Analyt von Interesse erkennen und an diesen binden kann,
wobei das Bindemittel, das speziell mindestens einige der Vielzahl von Bindemittelmolekülen erkennen und diese an den Nachweisträger binden kann, einen nachweisbaren Anteil umfasst.

14. Kit nach einem der Ansprüche 10 bis 13, wobei der Einfangträger ein Kügelchen ist,
oder wobei das Einfangträger-Bindemittel ein Antikörper oder Antikörperfragment ist, welcher/welches den Analyt von Interesse erkennen und sich an diesen binden kann.

15. Kit nach einem der Ansprüche 9 bis 14, wobei der Nachweisträger mehr als 1.000 für den Analyt von Interesse spezielle Bindemittelmoleküle umfasst;
wobei der Nachweisträger mehr als 10.000 Bindemittelmoleküle speziell für den Analyt von Interesse umfasst; oder wobei der Nachweisträger mehr als 100.000 Bindemittelmoleküle speziell für den Analyt von Interesse umfasst.

## Revendications

1. Un procédé permettant l'amplification de signaux consistant à :
ajouter à l'analyte d'intérêt un support de détection comprenant un support solide comprenant une pluralité de molécules d'un agent de liaison qui reconnaît et se lie à, l'analyte d'intérêt ; et
détecter au moins certaines molécules de la pluralité de molécules d'agent de liaison sur le support de détection en ajoutant un agent de liaison qui peut spécifiquement reconnaître, et se lier à, au moins certaines molécules de la pluralité de molécules de l'agent de liaison sur le support de détection.

2. Le procédé selon la revendication 1, dans lequel le support de détection est une bille ; ou dans lequel le support de détection comprend une pluralité de molécules d'une fraction détectable.

3. Le procédé selon l'une quelconque des revendications 1 à 2, consistant en outre à ajouter un support de capture, le support de capture comprenant un ou plusieurs agents de liaison de support de capture qui reconnaissent, et se lient à, l'analyte d'intérêt de façon à immobiliser l'analyte d'intérêt sur le support de capture ; de préférence
dans lequel l'analyte d'intérêt est lié au support de capture avant l'interaction avec le support de détection ; ou
dans lequel l'analyte d'intérêt est lié au support de capture après l'interaction avec le support de détection.

4. Le procédé selon l'une quelconque des revendications 1 à 3,
dans lequel l'agent de liaison qui peut spécifiquement reconnaître, et se lier à, au moins certaines molécules de la pluralité de molécules d'agent de liaison sur le support de détection comprend une fraction détectable,
dans lequel l'agent de liaison qui peut spécifiquement reconnaître, et se lier à, au moins certaines molécules de la pluralité de molécules d'agent de liaison sur le support de détection est un anticorps ou un fragment d'anticorps,
dans lequel l'agent de liaison qui peut spécifiquement reconnaître, et se lier à, au moins certaines molécules de la pluralité de molécules d'agent de liaison sur le support de détection ne reconnaît pas l'agent de liaison de capture utilisé pour lier l'analyte d'intérêt à un support solide de capture, ou
dans lequel l'agent de liaison qui peut spécifiquement reconnaître, et se lier à, au moins certaines molécules de la pluralité de molécules d'agent de liaison sur le support de détection comprend un agent de liaison soluble.

5. Le procédé selon l'une quelconque des revendications 3 à 4, dans lequel le support de capture est une bille.

6. Le procédé selon l'une quelconque des revendications 3 à 4, dans lequel l'agent de liaison fixé au support de capture est un anticorps ou un fragment d'anticorps qui peut reconnaître, et se lier à, l'analyte d'intérêt.

7. Le procédé selon l'une quelconque des revendications 1 à 6, dans lequel la pluralité de molécules de l'agent de liaison fixé au support de détection sont des anticorps ou des fragments d'anticorps qui reconnaissent et se lient à l'analyte d'intérêt.

8. Le procédé selon l'une quelconque des revendications 1 à 7, dans lequel le support de détection comprend plus de 10 000 molécules d'agent de liaison spécifiques à l'analyte d'intérêt, ou dans lequel le support de détection comprend plus de 100 000 molécules d'agent de liaison spécifiques à l'analyte d'intérêt.

9. Un kit de test d'un analyte d'intérêt comprenant : un support de détection, le support de détection comprenant un support solide comprenant une pluralité de molécules d'un agent de liaison qui peuvent reconnaître, et se lier à, l'analyte d'intérêt et un agent de liaison qui peut spécifiquement reconnaître, et se lier à, au moins certaines molécules de la pluralité de molécules d'agent de liaison sur le support de détection.

10. Le kit selon la revendication 9, comprenant en outre un support de capture, le support de capture comprenant un ou plusieurs agents de liaison de support de capture qui reconnaissent, et se lient à, l'analyte d'intérêt.

11. Le kit selon l'une quelconque des revendications 9 à 10, dans lequel le support de détection est une bille ; ou dans lequel le support de détection comprend une pluralité de molécules d'une fraction détectable.

12. Le kit selon l'une quelconque des revendications 9 à 11, dans lequel la pluralité de molécules d'agent de liaison fixées au support de détection sont des anticorps ou des fragments d'anticorps qui peuvent reconnaître, et se lier à, l'analyte d'intérêt.

13. Le kit selon l'une quelconque des revendications 9 à 10,
dans lequel l'agent de liaison qui peut spécifiquement reconnaître, et se lier à, au moins certaines molécules de la pluralité de molécules d'agent de liaison sur le support de détection comprend un agent de liaison soluble,
dans lequel l'agent de liaison qui peut spécifiquement reconnaître, et se lier à, au moins certaines molécules de la pluralité de molécules d'agent de liaison sur le support de détection est un anticorps ou un fragment d'anticorps qui peut reconnaître, et se lier à, l'analyte d'intérêt, ou
dans lequel l'agent de liaison qui peut spécifiquement reconnaître, et se lier à, au moins certaines molécules de la pluralité de molécules d'agent de liaison sur le support de détection comprend une fraction détectable.

14. Le kit selon l'une quelconque des revendications 10 à 13, dans lequel le support de capture est une bille, ou dans lequel l'agent de liaison de support de capture est un anticorps ou un fragment d'anticorps qui peut reconnaître, et se lier à, l'analyte d'intérêt.

15. Le kit selon l'une quelconque des revendications 9 à 14, dans lequel le support de détection comprend plus de 1 000 molécules d'agent de liaison spécifiques à l'analyte d'intérêt ;
dans lequel le support de détection comprend plus de 10 000 molécules d'agent de liaison spécifiques à l'analyte d'intérêt ; ou dans lequel le support de détection comprend plus de 100 000 molécules d'agent de liaison spécifiques à l'analyte d'intérêt.
